# EUROPEAN PATENT SPECIFICATION

(11) **EP 1 969 903 B1**
(45) Date of publication and mention of the grant of the patent: **24.03.2010**
(21) Application number: 06820445.2
(22) Date of filing: 06.12.2006
(51) Int. Cl.: H05B 33/08

(54) **IMPROVED LED ARRAY**
VERBESSERTES LED-ARRAY
MATRICE DE DEL AMELIOREE

(30) Priority: 06.12.2005 GB 0524909
(43) Date of publication of application: 17.09.2008
(73) Proprietor: Enfis Limited, Swansea Waterfront Swansea SA1 8PJ (GB)
(72) Inventor: JONES, Gareth, Mid Glamorgan CF72 9AJ (GB); BOARD, Kenneth, Swansea SA3 5EQ (GB); EVANS, Gareth, Peter, Swansea SA2 0LR (GB)
(74) Representative: Pearson, James Ginn
(86) International application number: PCT/GB2006/004571
(87) International publication number: WO 2007/066112

(56) References cited:
- EP-A- 1 152 642
- WO-A-02/099333
- US-A1- 5 684 523
- US-A1- 5 783 909

## Description

This invention relates to light-emitting diode (LED) arrays, including, but not limited to, arrangements for monitoring and controlling the power output of LEDs within an LED array.

As is well known in the art, the intensity of the output of an LED typically varies as the temperature of the LED varies. Generally, given a constant current power supply, the intensity of the output of an LED decreases with increasing temperature. A further change in output intensity (usually a reduction) is often found as an LED degrades with use, at a constant drive current.

In some applications, the intensity of light that is output by an LED is not critical and the expected power output, as indicated by the manufacturer, can be relied upon.
However, in many applications, it is important to know the power output that is being provided by a particular LED at any given time. By way of example, one application in which the power output of an LED array is likely to be important is the curing of composites in dental treatments.

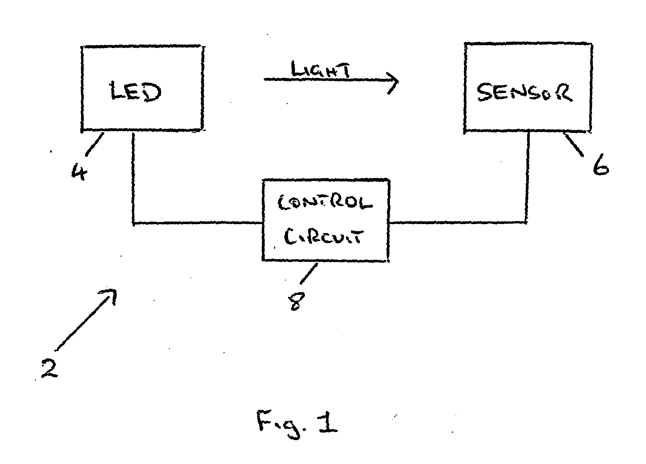

Figure 1 shows a monitoring system, indicated generally by the reference numeral 2, for monitoring the output of an LED. The monitoring system 2 comprises an LED 4, a photodiode sensor 6 remote from the LED, and a control circuit 8. In the use of the monitoring system 2, the output of the LED 4 is measured by the photodiode sensor 6 and data relating to that output is forwarded to the control circuit 8. In one exemplary application of the monitoring system 2, the input of the LED 4 can be controlled by the control unit 8 in order to obtain a desired output, as detected by the photodiode sensor 6.

The monitoring system 2 has the advantage of simplicity, but has a number of drawbacks. For example, the monitoring system 2 is unsuitable in certain applications where compactness of the LED device is important, for example when using LEDs to cure composites used in dental procedures or in skin treatments where the LED array is in close proximity to the treatment region. Also, the remote photodiode sensor will record extraneous light, such as background light and light emitted by other LEDs in an array (if any), which decreases the accuracy of the measurement.

In addition, photodetectors are generally sensitive to many wavelengths of light. Remotely monitoring an LED array which contains more than one emission wavelength from separate emitting LED chips within the LED array can lead to confusion between the light output measurements from each LED chip type. For example, a particular photodetector may be intended to detect light output from a first LED outputting light at a first wavelength, but may also detect light output from a second LED outputting light at a second wavelength that it is not intended to detect.

US Patent Number 5,684,523 discloses an optical line print head and an LED chip used therefor. The LED chip comprises a number of LEDs arranged to emit light in a first direction, and an LED arranged to emit light in a direction perpendicular to the first direction which is incident on a photodiode arranged to take measurements of the light. A cover member protects and shields the LED chips that make up the optical line print head. The light emitted in a first direction is passed through a lens array and the light emitted in the second direction is arranged so that it does not affect the light emitted in the first direction.

LED arrays have many applications, including curing composites or resins, for example as used in dental procedures, activating the whitening effect of gels used in dental treatments, antibacterial functions through the use of UV light, land the imaging of dental caries. Although the use of LED arrays for at least some of these purposes is known in the art, there are number of problems associated with known devices.

LED arrays are also enabling a number of new applications such as light sources for a variety of illumination applications such as entertainment lighting, architectural lighting, medical or dental lighting where the precise mixture of wavelengths (colours) and the intensity of these wavelengths is important and where compact low cost integrated approaches are desired. In such applications, having a compact, well controlled source of light of multiple wavelengths (colours) is often important. It may also be important to be able to dim the light and change the relative intensity of the different wavelengths as required. To perform such tasks effectively requires effective optical feedback. It should be noted that when mixing different wavelengths of light to provide a desired colour output, small variations in the intensity of a single coloured light source can have a significant effect on the overall colour representation of the mixed colour source.

In the exemplary applications mentioned above, it is often necessary to use multiple LEDs in order to achieve the required intensity of light. One approach for achieving this is to use large packaged LEDs; however, this renders the devices difficult to use in many applications, for example in dental applications where the devices must be used in the mouth of a patient. An alternative approach is to use smaller, lower power devices, but this tends to result in longer application times. This may not be attractive: for example, increasing treatment times when curing composites in the mouth of a dental patient would not be attractive. Moreover, in some applications, the desired effect (e.g. curing) may not be initiated unless a threshold power level is reached, even given very long exposure times.

In order to obtain high power density levels from LEDs, it is known to provide a large number of LEDs in a small area. Typically, LEDs covert about 5-20% of the electrical power they receive into light. Thus, typically, around 80-95% of the electrical energy received by an LED is dissipated as heat. Clearly, using large numbers of LEDs in a small area leads to large amounts of heat being generated, which must be handled in some way. Heat management systems used in such systems typically result in a significant increase in the overall size of such devices.

Another problem relating to the use of LED arrays in dental and other applications such as medical or industrial applications is complying with relevant regulations regarding permissible power levels. One approach is to calibrate an LED-array prior to use; however, such an arrangement does not enable changes in the power output of such a device to be detected whilst the device is in use. Thus, there is no way of checking whether or not the LED output increases, in use, to a level in excess of a safely limit. One solution to this problem is to take the device out of service from time-to-time in order to recalibrate the device; however this would not be acceptable in applications where taking the device out of service would be detrimental to the proper carrying out of the application. Another solution is to operate the device at a power level sufficiently below those permitted by the relevant regulations or safety considerations to ensure that the power output will not rise, in use, to an unsafe and/or unlawful level. Clearly, such an arrangement may reduce the effectiveness of the LED array.

The device and method of the present invention seeks to address at least some of the problems associated with the prior art systems and/or to provide alternatives to the devices and methods of the prior art.

The present invention provides an LED apparatus having an LED array wherein, the array comprises a plurality of LEDs and one or more optical sensors,
said one or more optical sensors are adjacent to one or more of said LEDs to form one or more LED-optical sensor pairs, each pair comprising an LED and an optical sensor, which is adjacent to the associated LED;
the optical sensor of each LED-optical sensor pair is arranged to measure the light output of the associated LED;
the LED apparatus comprises a frame that is adapted to partially cover said array of LEDs and optical sensors such that one or more of said LED-optical sensor pairs is at least partially shielded by said frame from light from any source other than the LED of the said LED-optical sensor pair; and
**characterised in that** the plurality of LEDs are mounted on the same side of one or more substrates such that the LEDs of the LED-optical sensor pairs transmit light in substantially the same direction as the remaining LEDs in the LED array and the partial shielding provided by the frame is such that some or all of the LEDs that form part of an LED-optical sensor pair are as arranged to provide part of the power output of the LED array.

Sources of extraneous light, from which the LED-optical sensor pair are at least partially shielded, may for example include light from other LEDs in the array and background light. In one form of the invention, one or more filters are provided, wherein said one or more filters are each associated with one of said LED-optical sensor pairs and each filter is arranged to attenuate light having a wavelength output different to that of the LED of the respective LED-optical sensor pair with which it is associated.

As noted above, some forms of the invention include one or more filters. By using filters, extraneous light having a wavelength other than that of the LED of the LED-photodiode pair can be attenuated before reaching the photodiode. The use of a filter is particular advantageous when not all of the LEDs in the array output light have the same wavelength or when the output of the LED of the LED-photodiode pair is off or emitting a low intensity light. In some forms of the invention, the or each filter is integrated onto the surface of the optical sensor with which it is associated. In an alternative arrangement, discrete filters are positioned between the LED and the optical sensor of the relevant LED-optical sensor pair.

In one form of the invention, the optical sensors are integrated.with the LEDs of the LED array so that the LEDs of the array and the optical sensors are provided in the same package. In another form of the invention, the optical sensors and LEDs of the array are provided as part of the same monolithic device.

In one form of the invention, the optical sensor is a photodiode. The skilled person would be aware, however, of other suitable optical sensors; no further details of suitable optical sensors will be given here.

In forms of the invention including shielding, the shielding may take many different forms. For example, screens may be used. Those screens may partially shield the relevant LED-optical sensor pairs, or may totally shield them, such that the light output by that LED does not exit the shielded area. In one form of the invention, the optical sensor is entirely screened from the remainder of the device with the exception of an opening that allows light from an individual LED to pass to the optical sensor.
Alternatively, or in addition, the LED-optical sensor pairs may be located in a sunken position relative to the remainder of said array. Alternatively or in addition, the photodiode may be selected so that it has a thinner substrate than the circuit elements around it such that the photodiode is located below the LEDs in the array so that the amount of light received from LEDs in the array other than the LED with which it is paired is reduced.
Alternatively, or in addition, the LED-optical sensor pairs may be capped. It should be noted that, in some forms of the invention, not all of the LED-optical sensor pairs are shielded to the same extent; indeed, one or more of the LED-optical sensor pairs may not be shielded at all.

As mentioned above, the plurality of LEDs are mounted on the same side of one or more substrates such that the LEDs of the LED-optical sensor pairs transit light in substantially the same direction as the remaining LEDs in the array. Further, the optical sensors may be mounted on the same side of said one or more substrates as said plurality of LEDs. In an alternative arrangement, a frame is provided onto which the optical sensors are mounted, wherein, in use, the frame is mounted to the LED array such that the optical sensors are located close to the LEDs with which they are associated.

The LED array may comprise a control circuit for controlling an output power of at least some of said LEDs on the basis of the output of at least one of said optical sensors. The control circuit may monitor the output of LEDs in the array. This monitoring may be carried out continuously, on a periodic basis, or from time-to-time as and when a control algorithm determines that monitoring is required. Clearly, the more often monitoring is carried out, the more accurately the LED output can match a desired output; however, monitoring less often reduces the computational overheads associated with the LED array.

The LEDs may include one or more groups of LEDs. Each of said groups of LEDs may include at least one LED that forms part of an LED-optical sensor pair. In an alternative embodiment, some, but not all, of the said groups of LEDs may include at least one LED that forms part of an LED-optical sensor pair.

In one form of the invention, each LED within a group is substantially identical. In such an arrangement, if all of the LEDs within a group have the same or a similar input current, then the outputs of each LED should be substantially the same. Accordingly, measuring the output of one LED in such a group (for example using an optical sensor) gives a measure indicative of the output of each of the LEDs in the group.

In one form of the invention, the output of fewer than all of the LEDs in a group is measured, and the input of each LED in the group is controlled on the basis of the said measurements. In one embodiment, the output of just one LED in a group is measured and the input of each LED is the group is controlled on the basis of that one measurement.

In one exemplary form of the invention, groups of LEDs are arranged in columns; however, other arrangements of groups of LEDs could be provided. LEDs within a group could be scattered around the LED array in any configuration.

In one form of the invention, each LED within each group of LEDs is identical to the others in the group and is driven by the same or a similar current to the others in the group, but the LEDs within different group may differ and/or may be driven by different currents. Thus, a plurality of groups of LEDs may be provided, with each group outputting different wavelengths and/or intensities of light, thereby enabling a light delivery regime to be accurately tailored and/or modified during use. Such an array has great flexibility.

One or more of said optical sensors may be associated with one or more integrators to enable the energy output by one or more of said LEDs over time to be determined. In this context, an integrator is a device arranged to determine the amount of light energy output by an LED in the array on the basis of the energy profile as detected by an optical sensor. For example, in a digital device, the energy output can be determined from discrete power output measurements together with data relating to the time intervals between readings. The skilled person would also be aware that an analogue integrator could be used. By making use of an integrator, the light energy output by a particular LED or group of LEDs in a given time period can be determined. Such an arrangement can be useful for treatment applications where the overall light dosage is of more significance than the peak power of the light energy.

The LED array may be provided with a cooling arrangement. Exemplary cooling arrangements include a heat sink, a heat pipe, an evaporative cooling system, or direct fluidic or air cooling.

The LED array may include one or more safety features. For example, a safety monitor may be provided such that, should the output power of one or more LEDs exceed a predetermined safety level, the input to that LED is reduced. A temperature sensor could be used, for example, to monitor the temperature of one or more of the LEDs so that the power input to one or more LEDs could be reduced if the temperature exceeded a predetermined temperature.

The present invention also provides a light delivery device comprising an LED array as set out above. The said LED array may be provided at the end of a finger, for example a flexible finger. Alternatively, or in addition, light from said LED array may be routed via a light guide. The present invention is well suited for installation as the LED for a light guide based delivery system since it allows for the control of the LED light intensity and therefore enables relatively high efficiency in the coupling of the light to the light guide when compared to systems that use external photodiodes as a means for monitoring the light intensity.

The device may be tiled together with similar or other devices in order to form a larger light emitting device.

The device may comprise a cooling arrangement for cooling the LEDs in said array. Exemplary cooling arrangements include a heat sink, a heat pipe, an evaporative cooling system or direct fluidic or air cooling.

The present invention further provides a feedback arrangement for use in controlling the outputs of a plurality of LEDs, wherein one or more of said LEDs is associated with an optical sensor to form an LED-optical sensor pair, wherein the optical sensor of each LED-optical sensor pair is arranged to measure the light output of the associated LED, the arrangement comprising a control circuit for controlling an output power of at least some of said LEDs on the basis of the outputs of said one or more optical sensors.

The said LEDs may include one or more groups of LEDs. Each group may include at least one LED that forms part of an LED-optical sensor pair. Each LED within a group may be substantially identical.

In one form of the invention, the feedback system measures the output of one or more LEDs in a group (for example just one LED in the group) and determines that the other LEDs in the group have the same performance. The outputs of all LEDs in the group are controlled in the same manner and the output of the or each LED in the group that is being measured is monitored until it has a desired output. It is then assumed that all the LEDs in the group have the said desired output.

In one form of the invention, the feedback arrangement includes one or more integrators to enable the energy output by one or more of said LEDs over time to be determined.

LED arrays and light delivery devices in accordance with the present invention have many uses, including, but not limited to, curing composites and resins, for example as used in dental procedures or industrial procedures such as the curing of resins in the manufacture of CDs and DVDs, activating the whitening effects of gels used in dental treatments, anti-bacterial, anti-fungal, anti-viral or anti-parasitic functions and the like, automotive illumination applications, signalling applications such as traffic lights, entertainment lighting, architectural lighting and any form of lighting where the precise mixture of wavelengths (colours) and the intensity of these wavelengths is important and/or where compact low-cost integrated approaches are needed.

Devices and methods in accordance with the invention will now be described, by way of example only, with reference to the accompanying schematic drawings in which:
Fig. 1 shows a known monitoring system for monitoring the output of an LED;
Fig. 2 is a perspective view of an LED array described by way of background to the present invention;
Fig. 2a is a perspective view of a variant of the LED array of Fig. 2 in accordance with an aspect of the present invention;
Fig. 3 is a plan view of the LED array of Fig. 2;
Fig. 4 is a block diagram of an exemplary control system for the LED arrays of Figs. 2, 2a and 3;
Fig. 5 is a side view of a light delivery system making use of an LED array of the present invention;
Fig. 6 shows a variant of the system of Fig. 5;
Fig. 7 is a plan view of an LED array in accordance with an aspect of the present invention;
Fig. 8 is plan view of an LED array in accordance with an aspect of the present invention; and
Fig. 9 is a perspective view of a frame used in an embodiment of the present invention.

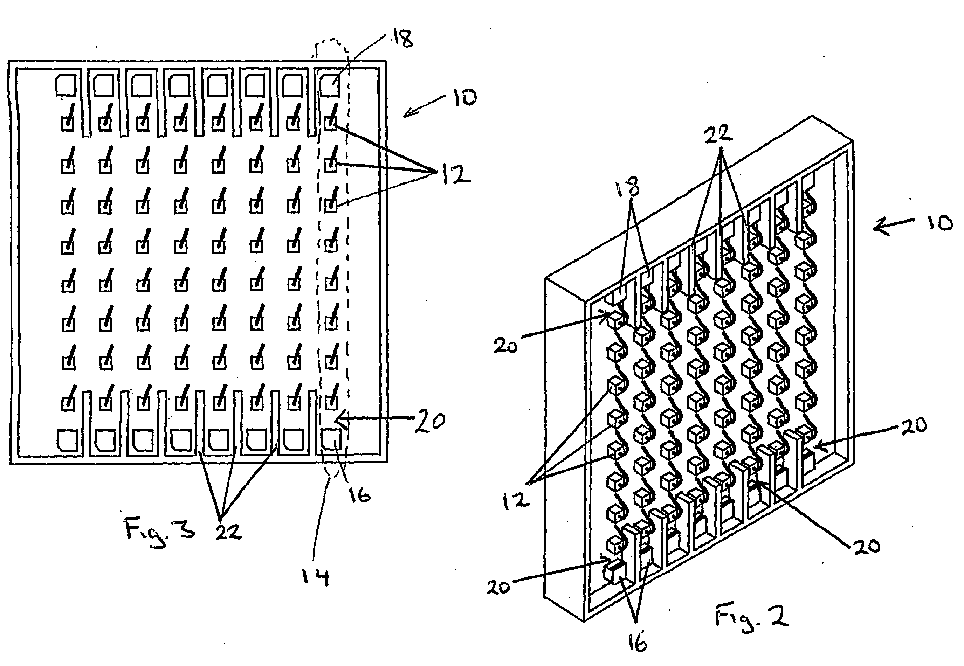

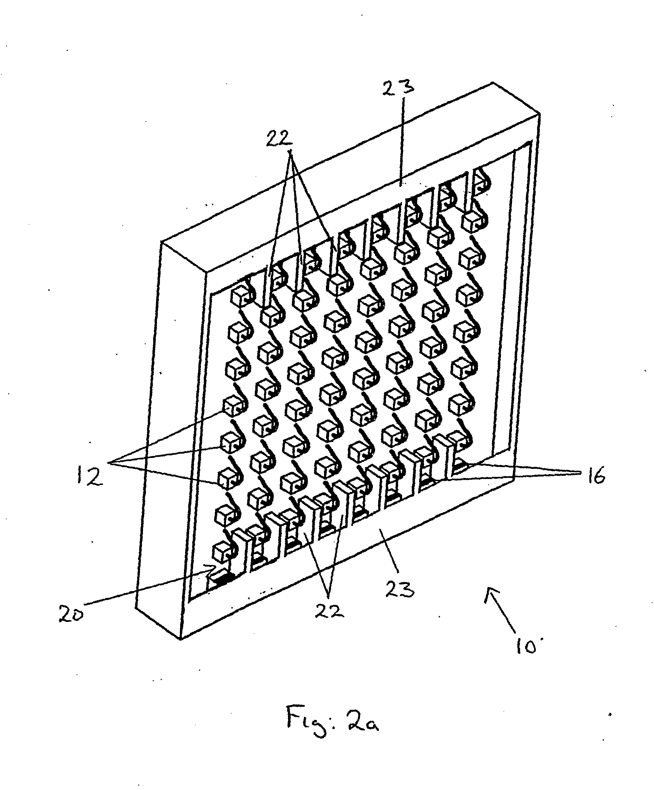

Figures 2 and 3 show an LED array, indicated generally by the reference numeral 10, that will now be described, by way of background to the present invention.

The array 10 comprises 64 light emitting diodes (LEDs) 12 arranged in 8 columns of 8 LEDs. By way of example, one column is indicated by the reference numeral 14. In one form of the array, each LED in a particular column is identical, and is provided with a similar or the same input current, so that each LED in a column should have the same power and wavelength output. In this form of the array, the LEDs of different columns can be different and/or may be provided with different inputs so that different columns may provide light outputs having different wavelengths and/or power outputs.

For example, the LED array 10 may have columns that output red, blue, green and ultraviolet light together with further colours which may be chosen to improve the light quality given out by the array. For example, when producing white light from the array, the further colour may be amber or yellow.

As shown in Figures 2 and 3, each column of LEDs is provided with a first 16 and a second 18 photodiode sensor. Each photodiode sensor is paired with one of the LEDs of the column to form an LED-photodiode pair, indicated generally by the reference numeral 20. If each of the LEDs in a column 14 is intended to be identical and has a similar or the same input current, each photodiode sensor effectively provides a measure of the output of each LED in a column.

Each LED-photodiode pair 20 is shielded from the other LEDs in the array 10 using a partial screen 22, in order to reduce the amount of light detected by the photodiode other than that emitted by the LED with which the photodiode is paired.

Although a plurality of screens 22 are shown in the example of Figures 2 and 3, other forms of shielding may be provided instead of, or in addition to, the use of screens. For example, the LED-photodiode pairs 20 may be capped, or may be located in a sunken position with respect to the other LEDs in the array 10. Clearly, the more completely an LED-photodiode pair is shielded from the other LEDs in the array, the more accurately the photodiode output will depend on the output of the LED it is intended to measure.

In addition to or instead of providing the LED-photodiode pair in a sunken position, the photodiode may be selected so that it has a thinner substrate than the circuit elements around it such that the photodiode is located in a lower position that the LEDs in the array. This has the effect of reducing the amount of light received by the photodiode from LEDs in the array other than the LED with which it is paired.

Figure 2a shows a variant (in accordance with the present invention), indicated generally by the reference numeral 10', of the LED array 10 of Figures 2 and 3. The array 10' is identical to the array 10 with the exception of the screening arrangement. The array 10' includes a partial screen 22, as in the array 10. The array 10' further comprises additional screens 23 that cover each of the photodiodes 16 and 18. The shielding provided by the array 10' is therefore more extensive than that provided by the array 10.

It will be apparent to the skilled person that the screening arrangements shown in Figure 2 and 2a could be adapted in a number of ways. For example, the screen 23 could be extended to cover the LEDs associated with the photodiodes 16 and 18. Although this arrangement would further increase the effectiveness of the screening, it would also reduce the effective power output of the LED array by preventing light from the LEDs associated with the photodiodes 16 and 18 from being used as part of the power output of the array.

In the example described above with reference to Figures 2 and 3, the LEDs of the array are arranged in columns of substantially identical LEDs. However, the identical LEDs need not be arranged in columns; sets of identical LEDs could be arranged,in any pattern in the array.

Further, as described above with reference to Figures 2 and 3, each set of LEDs comprises 8 LEDs, including 2 LED-photodiode pairs, with the sets of LEDs being arranged in columns. Of course, a different number of photodiodes per set of LEDs could be provided. If increased accuracy and certainty is required, then the number of photodiodes provided per set could be increased. Alternatively, the number of photodiodes provided per set could be reduced.
It should also be noted that the number of photodiodes provided does not need to be the same for each set of LEDs. Indeed, it is possible that at least some of the sets may not be provided with an LED-photodiode pair at all since it may be appropriate for some of the sets of LEDs to be used without being monitored.

Thus, in one form of the present invention, the LED array includes a number of sets of LEDs, with each LED in a particular set being substantially identical. At least some of those sets include one or more LEDs that are associated with a photodiode to form one or more LED-photodiode pairs; the outputs of the LED-photodiode pairs being indicative of the output of each LED in the set.

In order to verify the similarity of LEDs in a particular set, a calibration step may be added during the manufacturing stage.

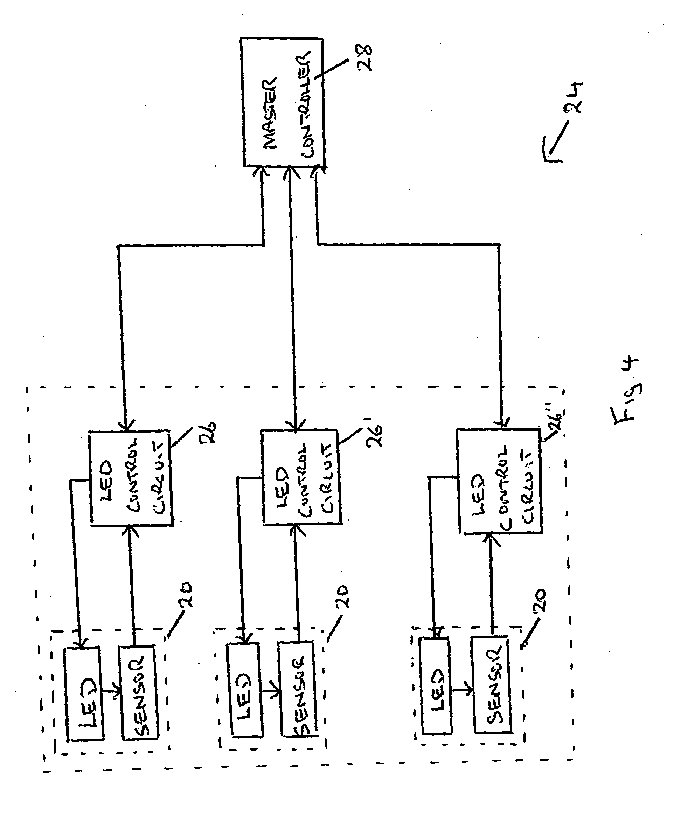

Each LED-photodiode pair 20 forms part of a feedback circuit used to control the array 10. Figure 4 is a block diagram, indicated generally by the reference numeral 24, of the control circuit for the array 10.

The circuit 24 includes a plurality of LED control circuits, with one control circuit being provided for each set of LEDs. For simplicity, the circuit 24 shows only three LED control circuits, labelled 26, 26' and 26" respectively. In addition, a master control circuit 28 provides control signals for each of the LED control circuits.

Each control circuit 26, 26', 26" provides control signals for the LEDs within its set. As described above, each photodiode measures the output of an LED, with that output being indicative of the output of each LED in the set; that measurement is sent to the relevant LED control circuit.
An algorithm in the relevant LED control unit compares the measured intensity with a desired intensity. If the measured intensity matches the desired intensity, then the LED control unit takes no action. Should the measured intensity not match the desired intensity then the LED control unit increases or decreases the current fed to the LED as is necessary to obtain the required output.

In one form of the invention, the feedback circuit operates on a periodic basis to ensure that the output of the LEDs is kept constant (or at a desired level, which level may not remain constant), by varying, as and when necessary, the current supplied to the LEDs. In another form of the invention, the feedback arrangement might be activated as and when a control circuits deems that a monitoring step is required. Clearly the smaller the time intervals between successive monitoring steps, the more accurately the LED output will match the desired output. Further, the monitoring could be made continuous by making use of analogue techniques. Of course, many control algorithms that could be used in the present invention will be apparent to the skilled person; no further details of possible control algorithms will be given here.

In one particular form of the invention, the temperature is also used as an input to the calculation of a first estimate of the current to be supplied to the LED. This is useful since one does not wish to overdrive the LEDs or to see any flicker when the LED is initially switched on. The efficiency of an LED is generally improved as the temperature decreases therefore an LED generally requires less current to output a given intensity of light when the temperature of the LED is low. Having the current input linked to the measured temperature allows the initial estimate of the required current to be close the current actually required. Once the LED is outputting light, the feedback arrangement described above can be used to adjust the current input, if required, in order to provide the required output in the manner described above.

As discussed above, in one form of the invention, each set of LEDs includes at least one LED-photodiode pair, and each LED-photodiode pair is shielded to at least partially prevent extraneous light from affecting the measurement taken. Such a system enables each set of LEDs to be independently monitored and controlled. Thus, if, for example, each set of LEDs outputs light has a different wavelength, then the overall output at each wavelength can be individually monitored and controlled.

The LED control units may also implement an algorithm in which the intensity of the output of the LEDs is integrated over time in order to obtain a measure of the light energy output by a given LED set in a given time (known as the "dosage" in certain applications). The integration may take the form of taking successive power output readings, assuming that the power output is constant between readings, multiplying each power reading by the time period over which it is assumed to be constant, and then summing the results over the time period over which the integration is being applied. Of course, the skilled person would be aware of a number of ways of carry out such an integration step; no further details will be given here.

The LED control units may be programmed such that the LEDs under their control output a certain, predetermined dosage of light via the LED array. In this manner, the photodiodes can be used as a monitor of light output rather than as a controlling feature, such that increases and decreases in the light output of the LEDs are used as an input to the calculation of the dosage. This is useful, for example, in medical treatments or curing of composites or resins where it is the total does of radiation rather than the exact peak level of the power that is important.

In the example of Figure 4, each of the LED control units 26, 26' and 26" is further controlled by a master control unit 28. The master control unit 28 can control the intensity of the light delivered by each set of LEDs, the period of time over which the LEDs are driven, and may include a pulsing algorithm, varying the intensity of the sets of LEDs and possibly switching between different wavelengths. The master control circuit 28 could also determine when a particular LED control circuit should check the outputs of one or more LEDs in an array. The particular composites or gel used in a process may have individual dosage profiles to optimise their curing or whitening. The use of such a master control unit 28 gives the LED array.a great deal of flexibility.

The LED control units may include a safety monitor such that should the output of an LED increase to a level in excess of a predetermined maximum permissible level, then the current input to that LED is reduced (or switched off), thereby enabling regulations setting a maximum power level to be complied with.

Furthermore, the LED control units may obtain data from one or more temperature sensors so that, should the temperature of any part of an LED array exceed a maximum permissible level, the power input to one or more of the sets of LEDs can be reduced (or switched off). This might be useful, for example, in dental applications where the LED array is intended to be inserted into a patient's mouth, since it would clearly be undesirable for the temperature of such an array to be allowed to reach a level at which the patient would become uncomfortable.

A temperature sensor may be used to allow an in-built calibration sensor system to control the LED power output more accurately and to adjust to the desired power levels more quickly.

In circumstances where the temperature dependence of the LEDs is strong, the system may be required to increase the current rapidly to the LEDs to keep the intensity constant as the heat sink temperature increases. However, when a maximum LED input current is reached, the system can change from adjusting the current to monitoring the light output so that an application such as a medical application that relies on the total dosage (rather than intensity) is able to be performed. This can lead to the production of lower cost systems where the control of the temperature is not so crucial to the application as the total dose of light is monitored.

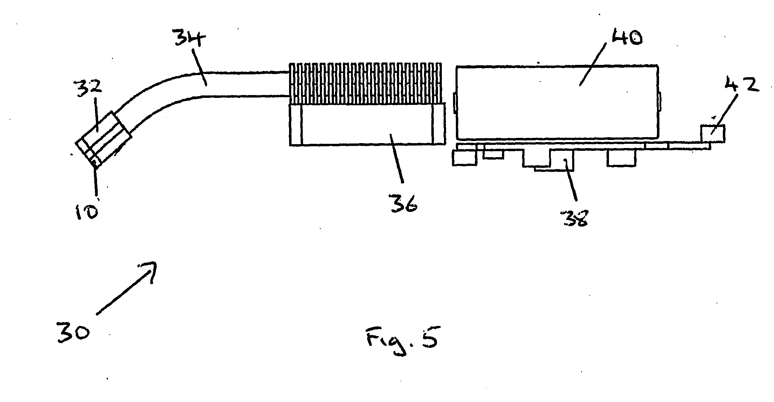

Figure 5 shows a light delivery system, indicated generally by the reference numeral 30, in accordance with an aspect of the present invention. The light delivery system 30 includes an LED array 10 having the form described above.

The LED array 10 is mounted on a copper mounting block 32, which is itself mounted to the end of a distal finger 34. The distal finger.may, for example, be suitable for insertion into the mouth of a patient. The distal finger 34 may be flexible in order to allow the array to be manipulated into many positions.

The distal finger is attached to a thermal management system 36 for removing heat from the LED array 10 to a location where the heat can be safely dispersed. The thermal management system could take one of many forms, such as a heat sink, a heat pipe, an evaporative cooling system, a direct fluidic or air cooling system, or a combination thereof. The skilled person would be able to implement any one of the above systems and would be aware of other thermal management systems that could be used. Accordingly, no further discussion of appropriate thermal management systems will be given here.

A printed circuit board 38 including control electronics is connected to the LED array 10 via the thermal management system 36, distal finger 34 and mounting block 32. The printed circuit board is also connected to a battery 40 and has a connector 42 that can be used, for example, to provide an alternative power source or to enable the control electronics to be programmed remotely.

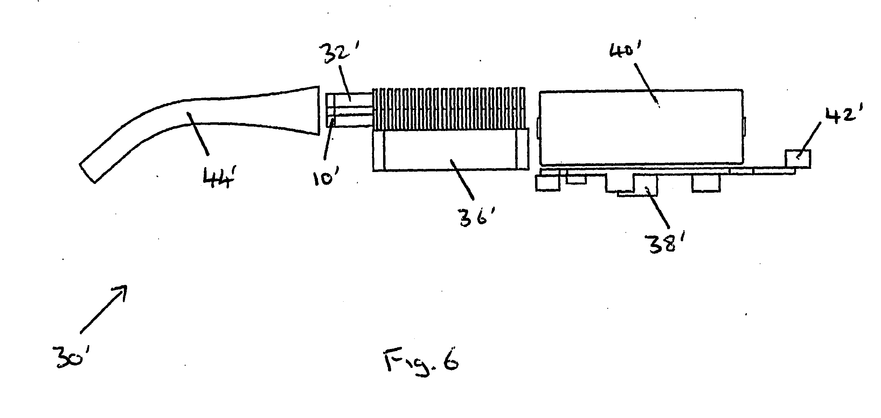

Figure 6 shows a light delivery system, indicated generally by the reference numeral 30', that is similar to the light delivery system 30 described above. The light delivery system 30' includes an LED array 10', copper mounting block 32', thermal management system 36', printed circuit board 38', battery 40' and connector 42' that are the same as or similar to the corresponding features of the system 30 described above. The mounting block 32' is coupled directly to the thermal management system 36', with the distal finger 34 being omitted. A light guide 44' is provided for routing the light output of the LED array 10' as required.

The light delivery system 30' might be appropriate in circumstances where it was not appropriate to have the LED array at the extreme end of the apparatus, for example due to safety concerns regarding the heat generated by the array.

The light guide 44' could take many forms. Indeed, a number of interchangeable light guides, each being designed for a particular application, could be provided.

In a variant of the present invention, the light array is provided in the form of a mouth guard that, in use, is placed over the teeth of a patient in order to expose a large area to a desired light radiation dose. Such a mouth guard may, for example, be used for curing, whitening or antibacterial applications of the forms described further above.

The invention has generally been described with reference to medical and dental applications; however, the invention is not so limited. For example, the LED array of the invention can be used in many lighting applications, including, but not limited to, entertainment lighting, architectural lighting, automotive illumination applications and signalling applications such as traffic light signals.

The present invention, insofar as is relates to medical and dental application can be arranged to be suitable for use by medical professionals, such as dentists and dental hygienists, as well as by untrained individuals in the home.

It should be noted that although an LED array comprising 64 LEDs in an 8x8 array has been described, the array could have any size. For example, the Applicant has produced an LED array including 1600 LEDs arranged in an 80x20 array, the array also including 20 photodiodes.

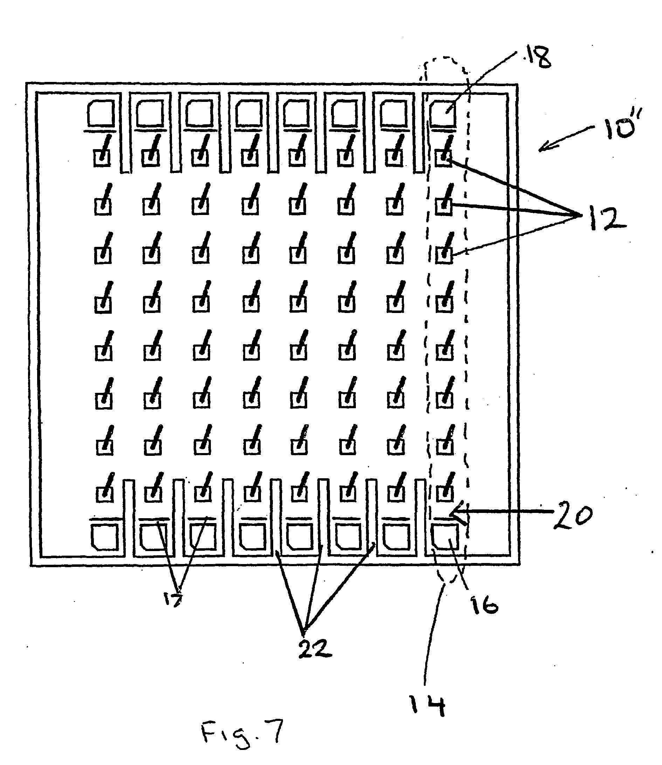

Figure 7 shows a variant, indicated generally by the reference numeral 10", of the LED array 10 of Figures 2, 2a and 3. The LED array 10" is identical to the array 10 with the exception that a filter 17 is provided between the LED and photodiode of each LED-photodiode pair 20. The filter passes light within a defined bandwidth that matches the light wavelength output by the corresponding LED. In this way, ambient light having a wavelength other than that of the LED of the LED-photodiode pair is attenuated before reaching the photodiode. The arrangement of Figure 7 is particular useful when not all of the LEDs in the array output light have the same wavelength. In that scenario, light having a different wavelength that is output by an LED close to the LED of the LED-photodiode pair that might otherwise impact on the signal output by the photodiode is attenuated. The arrangement of Figure 7 is also particularly useful when the output of the LED of the LED-photodiode pair is either emitting no light, or emitting a low intensity light since, in that scenario, extraneous light that reaches the photodiode could have a significant effect on the output of the photodiode.

In Figure 7, the filters are shown as discrete circuit elements positioned between an LED and a photodiode of an LED-photodiode pair. This arrangement is not essential. For example, the filters could be integrated onto the surface of the photodiodes.

In the examples described above with reference to Figures 2, 2a and 3, the LEDs of the array are arranged in columns of substantially identical LEDs. However, the identical LEDs need not be arranged in columns; sets of identical LEDs could be arranged in any pattern in the array.

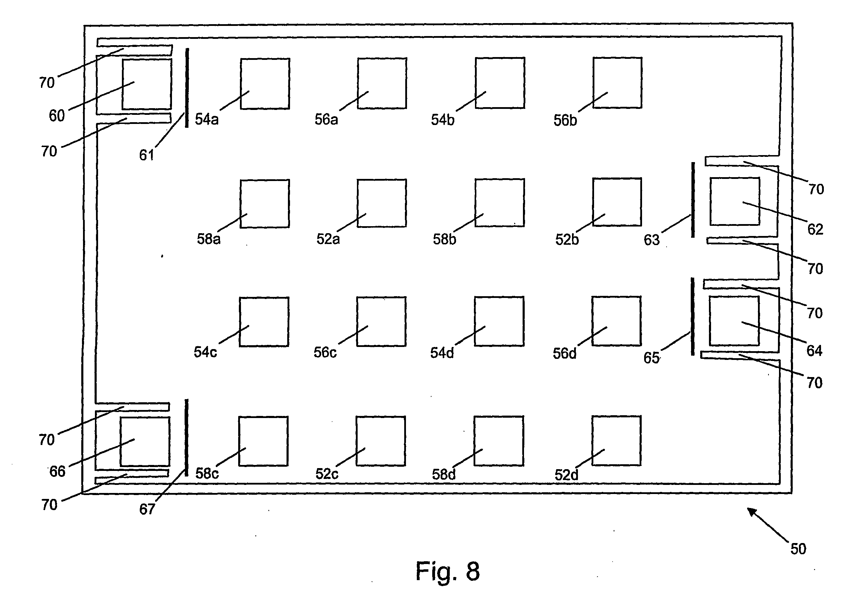

By way of example, Figure 8 is a plan view of an LED array, indicated generally by the reference numeral 50 in accordance with an embodiment of the invention. The LED array includes four groups of LEDs, each comprising four LEDs. The LEDs in each group are dispersed about the array. The first group of LEDs comprises red LEDs 52a, 52b, 52c and 52d. The second group of LEDs comprises green LEDs 54a, 54b, 54c and 54d. The third group of LEDs comprises blue LEDs 56a, 56b, 56c and 56d. The fourth group of LEDs comprises yellow LEDs 58a, 58b, 58c and 58d. The array 50 further comprises photodiodes 60, 62, 64 and 66 and filters 61, 63, 65 and 67 associated with the photodiodes 60, 62, 64 and 66 respectively.

Each group of LEDs in the array 50 includes an LED-photodiode pair. Specifically, LED 54a and photodiode 60, LED 52b and photodiode 62, LED 56d and photodiode 64 and LED 58c and photodiode 66 form first, second, third and fourth LED-photodiode pairs respectively. The LED array 50 also comprises partial shields 70 around the photodiodes of the LED-photodiode pairs.

In the use of the LED array 50, photodiodes 60, 62, 64 and 66 output a signal indicative of the level of green, red, blue and yellow light respectively. Those signals are provided to a controller, which controller controls the current supplied to the groups of LEDs in order to provide the desired light output.

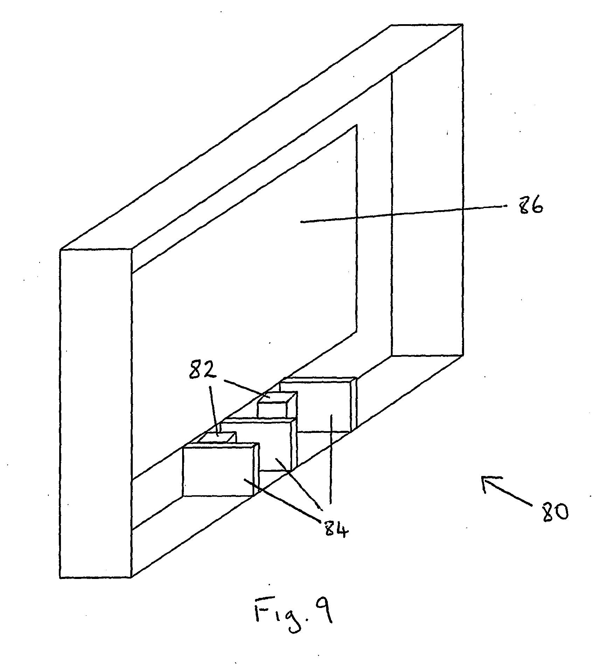

In the embodiments described above, the photodiodes have been located on the substrate as the LEDs in the array. This is not essential. For example, a frame could be provided for use in conjunction with the array, with the photodiodes being integrated in the frame. In use, the frame could be placed on top of the array such that the photodiodes are located adjacent to the LEDs the outputs of which they are intended to monitor. This arrangement may have the advantage of overcoming some problems associated with using an encapsulant which may trap bubbles into the LED array. In such an arrangement, the frame containing the photodiodes and the LED array may be stuck together, thereby providing an integrated LED-detector array. By way of example, Figure 9 shows an exemplary arrangement of a frame, indicated generally by the reference numeral 80, comprising a plurality of optical sensors 82 separated by shields 84. The frame forms a window 86 through which light from the LEDs of the array with which the frame 80 is intended to be used can pass. Of course, the shields 84 may take a different form, or may be omitted entirely, either because they are not required or because shields are provided by the LED array with which the frame is intended to be used. Figure 9 shows only two optical sensors 82; there could of course be many more optical sensors and those.sensors could be located in other places around the frame.

In one exemplary application, the LED array of the present invention could be used for liquid crystal display (LCD) backlighting. For example, groups of LEDs (such as red, green and blue LEDs) could be arranged in clusters, with each LED cluster being provided with optical monitoring. The clusters would be distributed across a large panel to form a large lighting panel.

In another application of the invention, the LED array could be used in conjunction with a phosphor coating used to convert the light output by the array into light having a different wavelength. Such an application could include filtering to filter out the light output by the phosphor coating such that the light output by the LED array itself can be monitored by the optical sensors.

## Claims

1. LED apparatus having an LED array (10) wherein, the array comprises a plurality of LEDs (12) and one or more optical sensors (16),
said one or more optical sensors (16, 18) are adjacent to one or more of said LEDs (12) to form one or more LED-optical sensor pairs (20), each pair comprising an LED and an optical sensor, which is adjacent to the associated LED;
the optical sensor (16, 18) of each LED-optical sensor pair (20) is arranged to measure the light output of the associated LED;
the LED apparatus comprises a frame that is adapted to partially cover said array of LEDs (12) and optical sensors (16) such that one or more of said LED-optical sensor pairs (20) is at least partially shielded by said frame from light from any source other than the LED of the said LED-optical sensor pair; and
**characterised in that** the plurality of LEDs are mounted on the same side of one or more substrates such that the LEDs of the LED-optical sensor pairs transmit light in substantially the same direction as the remaining LEDs in the LED array and the partial shielding provided by the frame is such that some or all of the LEDs (12) that form part of an LED-optical sensor pair (20) are arranged to provide part of the power output of the LED array.

2. LED apparatus as claimed in claim 1, further comprising one or more filters (17), wherein said one or more filters (17) are each associated with one of said LED-optical sensor pairs (20) and each filter (17) is arranged to attenuate light having a wavelength output different to that of the LED of the respective LED-optical sensor pair (20) with which it is associated.

3. LED apparatus as claimed in claim 2, wherein the or each filter (17) is integrated onto the surface of the optical sensor (16, 18) with which it is associated.

4. LED apparatus as claimed in any one of claims 1 to 3, further comprising one or more screens (22, 23) to provide shielding.

5. LED apparatus as claimed in claim 4, wherein one or more of said screens (22, 23) is a partial screen.

6. LED apparatus as claimed in any preceding claim, wherein at least one of said LED-optical sensor pairs (20) is provided in a sunken position relative to the remainder of said LED array.

7. LED apparatus as claimed in any preceding claim, wherein said plurality of LEDs are mounted on the same side of one or more substrates.

8. LED apparatus as claimed in any preceding claim, wherein the apparatus further comprises a control circuit for controlling an output power of at least some of said LEDs on the basis of the output of at least one of said optical sensors.

9. LED apparatus as claimed in any preceding claim, wherein said LEDs include one or more groups of LEDs.

10. LED apparatus as claimed in claim 9, wherein each group includes at least one LED that forms part of an LED-optical sensor pair.

11. LED apparatus as claimed in claim 9 or claim 10, wherein each LED within a group is substantially identical.

12. LED apparatus as claimed in any one of claims 9 to 11, wherein the output of fewer than all of the LEDs in a group is measured, and the input of each LED in the group is controlled on the basis of the said measurements.

13. LED apparatus as claimed in any preceding claim, wherein one or more of said optical sensors (16, 18) are associated with one or more integrators to enable the energy output by one or more of said LEDs over time to be determined.

14. LED apparatus as claimed in any preceding claim, wherein different LEDs in the LED array output light having different wavelengths.

15. A light delivery device (30) comprising an LED apparatus according to any one of claims 1 to 14.

16. A device (30) as claimed in claim 15, wherein said LED apparatus is provided at the end of a flexible finger (34).

17. A device (30) as claimed in claim 15 or claim 16, where light from said LED apparatus is routed via a light guide

18. A device (30) as claimed in any one of claims 15 to 17, further comprising a cooling arrangement (36) for cooling the LEDs in said apparatus.

## Patentansprüche

1. LED-Vorrichtung mit einer LED-Anordnung (10), wobei die Anordnung mehrere LEDs (12) und einen oder mehrere optische Sensoren (16) aufweist,
der eine oder die mehreren optischen Sensoren (16, 18) zu einer oder mehreren der LEDs (12) benachbart sind, um ein oder mehrere LED-/optischer Sensor-Paare (20) zu bilden, wobei jedes Paar eine LED und einen optischen Sensor aufweist, der zur zugehörigen LED benachbart ist;
der optische Sensor (16, 18) jedes LED-/optischer Sensor-Paares (20) eingerichtet ist, die Lichtabgabe der zugehörigen LED zu messen;
die LED-Vorrichtung einen Rahmen aufweist, der eingerichtet ist, teilweise die Anordnung der LEDs (12) und der optischen Sensoren (16) abzudecken, so daß eines oder mehrere der LED-/optischer Sensor-Paare (20) mindestens teilweise durch den Rahmen vor Licht aus irgendeiner anderen Quelle als der LED des LED-/optischer Sensor-Paars abgeschirmt ist; und
**dadurch gekennzeichnet, daß** die mehreren LEDs auf derselben Seite eines oder mehrerer Substrate angebracht sind, so daß die LEDs der LED-/optischer Sensor-Paare Licht in im wesentlichen dieselbe Richtung wie die restlichen LEDs in der LED-Anordnung aussenden, und die durch den Rahmen bereitgestellte Teilabschirmung so gestaltet ist, daß einige oder alle der LEDs (12), die einen Teil eines LED-/optischer Sensor-Paars (20) bilden, eingerichtet sind, einen Teil der Ausgangsleistung der LED-Anordnung bereitzustellen.

2. LED-Vorrichtung nach Anspruch 1, die ferner einen oder mehrere Filter (17) aufweist, wobei der eine oder die mehreren Filter (17) jeweils mit einem der LED-/optischer Sensor-Paare (20) verbunden sind und jeder Filter (17) eingerichtet ist, Licht zu dämpfen, das eine Wellenlängenabgabe aufweist, die sich von jener der LED des jeweiligen LED-/optischer Sensor-Paars (20) unterscheidet, mit der sie verbunden ist.

3. LED-Vorrichtung nach Anspruch 2, wobei der oder jeder Filter (17) in die Oberfläche des optischen Sensors (16, 18) integriert ist, mit der er verbunden ist.

4. LED-Vorrichtung nach einem der Ansprüche 1 bis 3, die ferner eine oder mehrere Blenden (22, 23) aufweist, um eine Abschirmung bereitzustellen.

5. LED-Vorrichtung nach Anspruch 4, wobei eine oder mehrere der Blenden (22, 23) eine Teilblende ist.

6. LED-Vorrichtung nach einem der vorhergehenden Ansprüche, wobei mindestens eines der LED-/optischer Sensor-Paare (20) relativ zum Rest der LED-Anordnung in einer versenkten Position vorgesehen ist.

7. LED-Vorrichtung nach einem der vorhergehenden Ansprüche, wobei die mehreren LEDs auf derselben Seite eines oder mehrerer Substrate angebracht sind.

8. LED-Vorrichtung nach einem der vorhergehenden Ansprüche, wobei die Vorrichtung ferner eine Steuerschaltung zum Steuern einer Ausgangsleistung mindestens einiger der LEDs auf der Grundlage der Ausgangsleistung mindestens eines der optischen Sensoren aufweist.

9. LED-Vorrichtung nach einem der vorhergehenden Ansprüche, wobei die LEDs eine oder mehrere Gruppen von LEDs umfassen.

10. LED-Vorrichtung nach Anspruch 9, wobei jede Gruppe mindestens eine LED aufweist, die einen Teil eines LED-/optischer Sensor-Paares bildet.

11. LED-Vorrichtung nach Anspruch 9 oder 10, wobei jede LED innerhalb einer Gruppe im wesentlichen identisch ist.

12. LED-Vorrichtung nach einem der Ansprüche 9 bis 11, wobei die Ausgangsleistung von weniger als allen LEDs in einer Gruppe gemessen wird und die Eingangsleistung jeder LED in der Gruppe auf der Grundlage der Messungen gesteuert wird.

13. LED-Vorrichtung nach einem der vorhergehenden Ansprüche, wobei einer oder mehrere der optischen Sensoren (16, 18) mit einem oder mehreren Integratoren verbunden sind, um es zu ermöglichen, daß die Energieabgabe durch eine oder mehrere der LEDs im Zeitablauf bestimmt wird.

14. LED-Vorrichtung nach einem der vorhergehenden Ansprüche, wobei unterschiedliche LEDs in der LED-Anordnung Licht mit unterschiedlichen Wellenlängen abgeben.

15. Lichtabgabevorrichtung (30), die eine LED-Vorrichtung nach einem der Ansprüche 1 bis 14 aufweist.

16. Vorrichtung (30) nach Anspruch 15, wobei die LED-Vorrichtung am Ende eines flexiblen Fingers (34) vorgesehen ist.

17. Vorrichtung (30) nach Anspruch 15 oder 16, wobei Licht aus der LED-Vorrichtung über einen Lichtleiter geleitet wird.

18. Vorrichtung (30) nach einem der Ansprüche 15 bis 17, die ferner eine Kühlanordnung (36) zur Kühlung der LEDs in der Vorrichtung aufweist.

## Revendications

1. Appareil à DEL (*diodes électroluminescentes*) comportant un groupement de DEL (10) dans lequel le groupement comprend une pluralité de DEL (12) et un ou plusieurs capteurs optiques (16),
ledit un ou lesdits plusieurs capteurs optiques (16, 18) est/sont adjacents à une ou plusieurs desdites DEL (12) pour former une ou plusieurs paires (20) de DEL - capteur optique, chaque paire comprenant une DEL et un capteur optique, qui est adjacent à la DEL associée ;
le capteur optique (16, 18) de chaque paire (20) de DEL - capteur optique est agencé pour mesurer la sortie de lumière de la DEL associée ;
l'appareil à DEL comprend un bâti qui est apte à couvrir partiellement ledit groupement de DEL (12) et de capteurs optiques (16) de sorte qu'une ou plusieurs desdites paires (20) de DEL - capteur optique est/sont au moins partiellement protégées par ledit bâti contre de la lumière provenant de toute autre source que la DEL de ladite paire de DEL - capteur optique ; et
**caractérisé en ce que** les DEL sont montées du même côté d'un ou de plusieurs substrats de sorte que les DEL des paires de DEL - capteur optique émettent une lumière dans sensiblement la même direction que les DEL restantes du groupement de DEL et que la protection partielle fournie par le bâti est telle que tout ou partie des DEL (12) qui font partie d'une paire (20) de DEL - capteur optique sont agencées pour fournir une partie de la sortie de puissance du groupement de DEL.

2. Appareil à DEL selon la revendication 1, comprenant en outre un ou plusieurs filtres (17), dans lequel ledit un ou lesdits filtres (17) est/sont tous associés à l'une desdites paires (20) de DEL - capteur optique et chaque filtre (17) est agencé pour atténuer une lumière ayant une sortie de longueur d'onde différente de celle de la DEL de la paire (20) de DEL - capteur optique respective à laquelle il est associée.

3. Appareil à DEL selon la revendication 2, dans lequel le ou chaque filtre (17) est intégré dans la surface du capteur optique (16, 18) auquel il est associé.

4. Appareil à DEL selon l'une quelconque des revendications 1 à 3, comprenant en outre un ou plusieurs écrans (22, 23) servant à fournir une protection.

5. Appareil à DEL selon la revendication 4, dans lequel un ou plusieurs desdits écrans (22, 23) sont des écrans partiels.

6. Appareil à DEL selon l'une quelconque des revendications précédentes, dans lequel au moins l'une desdites paires (20) de DEL - capteur optique est disposée dans une position enfoncée par rapport au reste dudit groupement de DEL.

7. Appareil à DEL selon l'une quelconque des revendications précédentes, dans lequel lesdites DEL sont montées du même côté d'un ou de plusieurs substrats.

8. Appareil à DEL selon l'une quelconque des revendications précédentes, dans lequel l'appareil comprend en outre un circuit de commande destiné à commander une puissance de sortie d'au moins certaines desdites DEL sur la base de la sortie d'au moins l'un desdits capteurs optiques.

9. Appareil à DEL selon l'une quelconque des revendications précédentes, dans lequel lesdites DEL comprennent un ou plusieurs groupes de DEL.

10. Appareil à DEL selon la revendication 9, dans lequel chaque groupe inclut au moins une DEL qui fait partie d'une paire de DEL - capteur optique.

11. Appareil à DEL selon la revendication 9 ou la revendication 10, dans lequel toutes les DEL d'un groupe sont sensiblement identiques.

12. Appareil à DEL selon l'une quelconque des revendications 9 à 11, dans lequel la sortie de quelques DEL, mais pas de toutes les DEL, d'un groupe est mesurée, et l'entrée de chaque DEL du groupe est commandée sur la base desdites mesures.

13. Appareil à DEL selon l'une quelconque des revendications précédentes, dans lequel un ou plusieurs desdits capteurs optiques (16, 18) est/sont associés à un ou plusieurs intégrateurs pour permettre de déterminer dans le temps la sortie d'énergie d'une ou de plusieurs desdites DEL.

14. Appareil à DEL selon l'une quelconque des revendications précédentes, dans lequel des DEL différentes du groupement de DEL fournissent de la lumière ayant des longueurs d'onde différentes.

15. Dispositif de délivrance de lumière (30) comprenant un appareil à DEL selon l'une quelconque des revendications 1 à 14.

16. Dispositif (30) selon la revendication 15, dans lequel ledit appareil à DEL est disposé à l'extrémité d'un doigt souple (34),

17. Dispositif (30) selon la revendication 15 ou la revendication 16, dans lequel la lumière provenant dudit appareil à DEL est dirigée via un guide de lumière.

18. Dispositif (30) selon l'une quelconque des revendications 15 à 17, comprenant en outre un agencement de refroidissement (36) destiné à refroidir les DEL dudit appareil.
